# EUROPEAN PATENT SPECIFICATION

(11) **EP 4 105 324 B1**
(45) Date of publication and mention of the grant of the patent: **30.10.2024**
(21) Application number: 21823717.0
(22) Date of filing: 15.07.2021
(51) Int. Cl.: C12N 9/10, C12N 15/77, C12P 19/32

(54) **NOVEL BIFUNCTIONAL PYR OPERON TRANSCRIPTIONAL REGULATOR/URACIL PHOSPHORIBOSYLTRANSFERASE VARIANT, AND METHOD FOR PRODUCING IMP USING SAME**
NEUE BIFUNKTIONELLE PYR-OPERON-TRANSKRIPTIONSREGULATOR/URACILPHOSPHORIBOSYLTRANSFERASE-VARIANTE UND VERFAHREN ZUR HERSTELLUNG VON IMP UNTER VERWENDUNG DAVON
NOUVEAU VARIANT D'URACIL PHOSPHORIBOSYLTRANSFÉRASE/RÉGULATEUR DE TRANSCRIPTION DE L'OPÉRON PYR BIFONCTIONNEL ET PROCÉDÉ DE PRODUCTION D'IMP L'UTILISANT

(30) Priority: 29.04.2021 KR 20210055714
(43) Date of publication of application: 21.12.2022
(73) Proprietor: CJ Cheiljedang Corporation, Seoul 04560 (KR)
(72) Inventor: KWON, Nara, Seoul 04560 (KR); LEE, Ji Hye, Seoul 04560 (KR); RHO, Jin Ah, Seoul 04560 (KR); KWON, Hee Su, Seoul 04560 (KR); BONG, Hyun-Ju, Seoul 04560 (KR); SON, Sung Kwang, Seoul 04560 (KR); HUH, Lan, Seoul 04560 (KR); YOO, Hyeryun, Seoul 04560 (KR); KIM, Bina, Seoul 04560 (KR)
(74) Representative: Mewburn Ellis LLP
(86) International application number: PCT/KR2021/009105
(87) International publication number: WO 2022/231067

(56) References cited:
- KR-A- 20160 078 694
- KR-A- 880 011 346
- KR-B1- 101 916 611
- KR-B1- 102 277 410
- No further relevant documents disclosed
- DATABASE Protein 16 June 2019 (2019-06-16), ANONYMOUS: "bifunctional pyr operon transcriptional regulator/uracil phosphoribosyltransferase PyrR [Corynebacterium stationis]", XP055982401, retrieved from Genbank Database accession no. WP_079005494
- BAMPIDIS VASILEIOS, GIOVANNA AZIMONTI, MARIA DE LOURDES BASTOS, HENRIK CHRISTENSEN, BIRGIT DUSEMUND, MARYLINE KOUBA, MOJCA KOS DUR: "Safety and efficacy of IMP (disodium 5′-inosinate) produced by fermentation with Corynebacterium stationis KCCM 80161 for all animal species", EFSA JOURNAL, vol. 18, no. 5, 27 May 2020 (2020-05-27), pages e06140, XP055981488, DOI: 10.2903/j.efsa.2020.6140

## Description

### [Technical Field]

The present disclosure relates to a novel bifunctional pyr operon transcriptional regulator/uracil phosphoribosyltransferase variant, a *Corynebacterium stationis* strain comprising the variant, and a method for producing IMP using the strain.

### [Background Art]

Various studies are being conducted to develop highly efficient microorganisms and fermentation process technologies for the production of IMP and other useful substances. For example, a target substance-specific approach is mainly used in which the expression of a gene encoding an enzyme involved in IMP biosynthesis is increased, or in which genes unnecessary for the biosynthesis are removed (EP 3722430 A1, US 2020-0347346 A1). As an example of the enzyme involved in the IMP biosynthesis, there is a bifunctional pyr operon transcriptional regulator/uracil phosphoribosyltransferase (https://www.uniprot.org/uniprot/A0A0M2NXT 1).

However, it is still necessary to conduct studies to effectively increase the IMP producing ability as the demand for IMP increases.

### [Disclosure]

### [Technical Problem]

The present inventors have developed a novel bifunctional pyr operon transcriptional regulator/uracil phosphoribosyltransferase variant, a *Corynebacterium stationis* strain comprising the variant, and a method for producing IMP using the strain, thereby completing the present disclosure.

### [Technical Solution]

An object of the present disclosure is to provide a bifunctional pyr operon transcriptional regulator/uracil phosphoribosyltransferase variant consisting of an amino acid sequence represented by SEQ ID NO: 1, in which proline, which is an amino acid corresponding to position 108 of an amino acid sequence of SEQ ID NO: 3, is substituted with serine.

Another object of the present disclosure is to provide a polynucleotide encoding the variant of the present disclosure.

Still another object of the present disclosure is to provide a *Corynebacterium stationis* strain that comprises the variant of the present disclosure or a polynucleotide encoding the variant and has IMP producing ability.

Still another object of the present disclosure is to provide a method for producing IMP, which comprises culturing a *Corynebacterium stationis* strain that comprises a variant of the present disclosure or a polynucleotide encoding the variant and has IMP producing ability in a medium.

### [Advantageous Effects]

In the case of culturing a *Corynebacterium stationis* strain comprising the bifunctional pyr operon transcriptional regulator/uracil phosphoribosyltransferase variant of the present disclosure, it is possible to produce IMP at a higher yield as compared to the case of existing microorganisms having unmodified polypeptides.

### [Best Mode]

The present disclosure will be described in detail as follows. Meanwhile, each of the descriptions and embodiments disclosed in the present disclosure may be applied to other descriptions and embodiments. In other words, all combinations of various elements disclosed in the present disclosure belong to the scope of the present disclosure.
The present invention is as defined in the claims. This invention and other facets of the present disclosure may be more fully understood by reference to the following description.

An aspect of the present disclosure provides a bifunctional pyr operon transcriptional regulator/uracil phosphoribosyltransferase variant consisting of an amino acid sequence represented by SEQ ID NO: 1, in which proline, which is an amino acid corresponding to position 108 of an amino acid sequence of SEQ ID NO: 3, is substituted with serine.

The variant of the present disclosure may have, comprise, consist of, or consist essentially of the amino acid sequence represented by SEQ ID NO: 1.

In disclosed but not explicitly claimed variants of the present disclosure, the amino acid corresponding to position 108 based on the amino acid sequence of SEQ ID NO: 3 in the amino acid sequence represented by SEQ ID NO: 1 is serine, and may comprise an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1. It is apparent that variants having amino acid sequences in which some sequences are deleted, modified, substituted, conservatively substituted, or added are also included in the scope of the present disclosure as long as the amino acid sequences have such homology or identity and exhibit efficacy corresponding to that of the variant of the present disclosure.

Examples thereof include variants having sequence addition or deletion that do not alter the function of the variant of the present disclosure at the N-terminus, C-terminus of the amino acid sequence and/or inside the amino acid sequence, naturally occurring mutation, silent mutation, or conservative substitution.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of the proteins or polypeptides.

In the present disclosure, the term "variant" refers to a polypeptide which has an amino acid sequence different from that of the variant before being varied by conservative substitution and/or modification of one or more amino acids but maintains the functions or properties. Such a variant may generally be identified by modifying one or more amino acids of the amino acid sequence of the polypeptide and evaluating the properties of the modified polypeptide. In other words, the ability of the variant may be increased, unchanged, or decreased as compared to that of the polypeptide before being varied. Some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, mutant, mutein, and divergent and is not limited thereto as long as it is a term used with the meaning of variation. The claimed variants are polypeptides consisting of an amino acid sequence represented by SEQ ID NO: 1, in which proline, which is an amino acid corresponding to position 108 of the amino acid sequence of SEQ ID NO: 3, is substituted with serine.

The disclosed but not explicitly claimed variants of the disclosure may comprise deletions or additions of amino acids that have minimal effect on the properties and secondary structure of the polypeptide. For example, a signal (or leader) sequence that is co-translationally or post-translationally involved in the protein translocation may be conjugated to the N-terminus of the variant. The variant may be conjugated with other sequences or linkers so as to be identified, purified, or synthesized.

In the present disclosure, the term "homology" or "identity" means the degree of similarity between two given amino acid sequences or base sequences and may be expressed as a percentage. The terms homology and identity may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or a part of the sequence in moderately or highly stringent conditions. It is apparent that hybridization also includes hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al., (1988) Proc. Natl. Acad. Sci. USA 85:2444. Alternatively, the homology, similarity, or identity may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego,1994; and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al., (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

As an example of the present disclosure, the variant of the present disclosure may exhibit bifunctional pyr operon transcriptional regulator/uracil phosphoribosyltransferase activity. The variant of the present disclosure may exhibit activity so as to have increased IMP producing ability as compared to a wild-type polypeptide exhibiting bifunctional pyr operon transcriptional regulator/uracil phosphoribosyltransferase activity.

In the present disclosure, the term "Bifunctional pyr operon transcriptional regulator/uracil phosphoribosyltransferase" regulates the transcriptional attenuation of the pyrimidine nucleotide (pyr) operon in a uridine-dependent manner. Specifically, the "bifunctional pyr operon transcriptional regulator/uracil phosphoribosyltransferase" of the present disclosure may be used interchangeably with "PyrR". In the present disclosure, the sequence of the bifunctional pyr operon transcriptional regulator/uracil phosphoribosyltransferase can be obtained from GenBank of the NCBI, a known database. Specifically, the bifunctional pyr operon transcriptional regulator/uracil phosphoribosyltransferase may be a polypeptide having bifunctional pyr operon transcriptional regulator/uracil phosphoribosyltransferase activity encoded by the *pyrR* gene, but is not limited thereto.

In the present disclosure, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, "corresponding region" generally refers to a similar or corresponding position in a related protein or a reference protein.

For example, an arbitrary amino acid sequence is aligned with SEQ ID NO: 3, and based on this, each amino acid residue of the amino acid sequence may be numbered with reference to the amino acid residue of SEQ ID NO: 3 and the numerical position of the corresponding amino acid residue. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000, Trends Genet. 16:276-277), and the like may be used, but the program and algorithm are not limited thereto, and a sequence alignment program, a pairwise sequence comparison algorithm, and the like known in the art may be appropriately used.

Another aspect of the present disclosure is to provide a polynucleotide encoding the variant of the present disclosure.

In the present disclosure, the term "polynucleotide" is a DNA or RNA strand having a certain length or more as a polymer of nucleotides in which nucleotide monomers are connected in a long chain by covalent bonds, and more specifically means a polynucleotide fragment encoding the variant.

The claimed polynucleotides encoding the variant of the present invention encode the amino acid sequence represented by SEQ ID NO: 1. As an example of the present disclosure, the polynucleotide of the present disclosure may have or comprise the sequence of SEQ ID NO: 2. The polynucleotide of the present disclosure may consist of or consist essentially of the sequence of SEQ ID NO: 2.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Disclosed but not explicitly claimed polynucleotides of the present disclosure may have or comprise a base sequence having 70% or more, 75% or more, 80% or more, 85% or more, 90% or more, 95% or more, 96% or more, 97% or more, 98% or more, but less than 100% homology or identity to the sequence of SEQ ID NO: 2 or may consist of or consist essentially of a base sequence having 70% or more, 75% or more, 80% or more, 85% or more, 90% or more, 95% or more, 96% or more, 97% or more, 98% or more, but less than 100% homology or identity to the sequence of SEQ ID NO: 2, but is not limited thereto. Here, in the sequence having homology or identity, the codon encoding the amino acid corresponding to position 108 of SEQ ID NO: 1 may be one of the codons encoding serine.

The polynucleotide of the present disclosure may comprise a probe that can be prepared from a known gene sequence, for example, a sequence without limitation as long as it is a sequence that can hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, namely polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed one time, specifically two to three times at a salt concentration and temperature equivalent to 60°C, 1X SSC, 0.1% SDS, specifically 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tₘ value of 55°C and the above-described conditions. The Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (for example, J. Sambrook et al., Molecular Cloning, A Laboratory Manual, 2nd Edition, Cold Spring Harbor Laboratory press, Cold Spring Harbor, New York, 1989).

Another aspect of the present disclosure is to provide a vector comprising the polynucleotide of the present disclosure. The vector may be an expression vector for expressing the polynucleotide in a host cell, but is not limited thereto.

The vector of the present disclosure may include a DNA construct comprising a polynucleotide sequence encoding a polypeptide of interest operably linked to a suitable expression control region (or expression control sequence) so that the polypeptide of interest may be expressed in a suitable host. The expression control region may contain a promoter capable of initiating transcription, any operator sequence for controlling the transcription, a sequence encoding a suitable mRNA ribosome binding site, and a sequence controlling termination of transcription and translation. The vector may be transformed into a suitable host cell and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, and the like may be used as a phage vector or a cosmid vector, and pDZ system, pBR system, pUC system, pBluescript II system, pGEM system, pTZ system, pCL system, pET system, and the like may be used as a plasmid vector. Specifically, pDZ, pDC, pDCM2, pACYC177, pACYC184, pCL, pECCG117, pUC19, pBR322, pMW118, and pCC1BAC vectors and the like may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further contain a selection marker for the confirmation of chromosome insertion. The selection marker is for selecting the cells transformed with vectors, that is, for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells can be selected.

In the present disclosure, the term "transformation" means that a vector comprising a polynucleotide encoding a target polypeptide is introduced into a host cell or a microorganism so that the polypeptide encoded by the polynucleotide can be expressed in the host cell. The transformed polynucleotide may be located by being inserted into the chromosome of the host cell or located outside the chromosome as long as it can be expressed in the host cell. The polynucleotide comprises DNA and/or RNA encoding a polypeptide of interest. The polynucleotide may be introduced in any form as long as it can be introduced into a host cell and expressed. For example, the polynucleotide may be introduced into a host cell in the form of an expression cassette, which is a gene construct containing all elements required for self-expression. The expression cassette may usually contain a promoter operably linked to the polynucleotide, a transcription termination signal, a ribosome binding site, and a translation termination signal. The expression cassette may be in the form of an expression vector capable of self-replicating. The polynucleotide may be introduced into a host cell in its own form and operably linked to a sequence required for expression in the host cell, but is not limited thereto.

In the above, the term "operably linked" means that the polynucleotide sequence is functionally linked to a promoter sequence that initiates and mediates transcription of the polynucleotide encoding the variant of interest of the present disclosure.

Still another aspect of the present disclosure is to provide a *Corynebacterium stationis* strain that comprises the variant of the present disclosure or the polynucleotide of the present disclosure.

The strain of the present disclosure may comprise a modified polypeptide of the present disclosure, a polynucleotide encoding the polypeptide, or a vector comprising the polynucleotide of the present disclosure.

In the present disclosure, the "strain (or microorganism)" includes all wild-type microorganisms or naturally or artificially genetically modified microorganisms, and it may be a microorganism in which a specific mechanism is weakened or strengthened due to a insertion of an external gene or an activity enhancement or inactivation of an endogenous gene, and it may be a microorganism comprising genetic modification for production of a polypeptide, protein, or product of interest.

The strain of the present disclosure may be a strain comprising any one or more of the variant of the present disclosure, the polynucleotide of the present disclosure, or a vector comprising the polynucleotide of the present disclosure; a strain modified to express the variant of the present disclosure or the polynucleotide of the present disclosure; a strain (for example, a recombinant strain) expressing the variant of the present disclosure or the polynucleotide of the present disclosure; or a strain (for example, a recombinant strain) exhibiting the activity of the variant of the present disclosure, but is not limited thereto.

The strain of the present disclosure may be a strain having 5'-inosine monophosphate (IMP) producing ability.

The strain of the present disclosure may be a microorganism naturally having bifunctional pyr operon transcriptional regulator/uracil phosphoribosyltransferase or IMP producing ability or a microorganism in which the variant of the present disclosure or a polynucleotide encoding the variant (or a vector comprising the polynucleotide) is introduced into a parent strain that does not have bifunctional pyr operon transcriptional regulator/uracil phosphoribosyltransferase or IMP producing ability and/or in which IMP producing ability is conferred on the parent strain, but is not limited thereto.

For example, the strain of the present disclosure is a cell or microorganism that is transformed with a vector comprising the polynucleotide of the present disclosure or a polynucleotide encoding the variant of the present disclosure and expresses the variant of the present disclosure. For the purposes of the present disclosure, the strain of the present disclosure may include all microorganisms that comprise the variant of the present disclosure and can produce IMP. For example, the strain of the present disclosure may be a recombinant strain in which a polynucleotide encoding the variant of the present disclosure is introduced into a natural wild-type microorganism or a microorganism producing IMP to thus express a bifunctional pyr operon transcriptional regulator/uracil phosphoribosyltransferase variant and having increased IMP producing ability. The recombinant strain having increased IMP producing ability may be a microorganism having increased IMP producing ability as compared to a natural wild-type microorganism or a bifunctional pyr operon transcriptional regulator/uracil phosphoribosyltransferase unmodified microorganism (namely, a microorganism expressing wild-type bifunctional pyr operon transcriptional regulator/uracil phosphoribosyltransferase (SEQ ID NO: 3) or a microorganism that does not express modified (SEQ ID NO: 1) protein), but is not limited thereto. For example, the strain having increased IMP producing ability of the present disclosure may be a microorganism having increased IMP producing ability as compared to *Corynebacterium stationis* which comprises a polypeptide of SEQ ID NO: 3 or a polynucleotide encoding the polypeptide, but is not limited thereto. As an example, the bifunctional pyr operon transcriptional regulator/uracil phosphoribosyltransferase unmodified microorganism that is a target strain for comparison of the increase in IMP producing ability may be an CJI2332 strain (KCCM12277P, KR 10-1950141 B1), but is not limited thereto.

For example, the recombinant strain having increased producing ability may have IMP producing ability increased by about 1% or more, specifically about 1% or more, about 2.5% or more, about 5% or more, about 6% or more, about 7% or more, about 8% or more, about 9% or more, about 10% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 20.5% or more, about 21% or more, about 21.5% or more, about 22% or more, about 22.5% or more, about 23% or more, about 23.5% or more, about 24% or more, about 24.5% or more, about 25% or more, about 26% or more, about 27% or more, about 27.5% or more, about 27.6% or more, about 27.7% or more, about 27.8% or more, or about 27.9% or more (the upper limit is not particularly limited and may be, for example, about 200% or less, about 150% or less, about 100% or less, about 50% or less, about 40% or less, or about 30% or less) as compared to the IMP producing ability of the parent strain before being varied or an unmodified microorganism, but the increased amount is not limited thereto as long as the producing ability has an increased amount of a + value as compared to the producing ability of the parent strain before being varied or an unmodified microorganism. In another example, the recombinant strain having increased producing ability may have IMP producing ability increased by about 1.01 times or more, about 1.02 times or more, about 1.03 times or more, about 1.05 times or more, about 1.06 times or more, about 1.07 times or more, about 1.08 times or more, about 1.09 times or more, about 1.10 times or more, about 1.11 times or more, about 1.12 times or more, about 1.13 times or more, about 1.14 times or more, about 1.15 times or more, about 1.16 times or more, about 1.17 times or more, about 1.18 times or more, about 1.19 times or more, about 1.20 times or more, about 1.21 times or more, about 1.22 times or more, about 1.23 times or more, about 1.24 times or more, about 1.25 times or more, about 1.26 times or more, about 1.27 times or more, or about 1.28 times or more (the upper limit is not particularly limited and may be, for example, about 10 times or less, about 5 times or less, about 3 times or less, or about 2 times or less) as compared to the IMP producing ability of the parent strain before being varied or an unmodified microorganism, but the rate of increase is not limited thereto. The term "about" is a range including all of ±0.5, ±0.4, ±0.3, ±0.2, ±0.1, and the like, includes all values in a range equal to or similar to the value following the term "about", but is not limited thereto.

In the present disclosure, the "unmodified microorganism" does not exclude strains comprising mutation that may occur naturally in microorganisms, and may be a wild-type strain or a natural strain itself or may be a strain before the trait is changed by genetic variation due to natural or artificial factors. For example, the unmodified microorganism may be a strain into which the bifunctional pyr operon transcriptional regulator/uracil phosphoribosyltransferase variant described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

The claimed microorganisms are *Corynebacterium stationis* strains. Other disclosed but not explicitly claimed microorganisms of the present disclosure may be *Corynebacterium stationis*, *Corynebacterium glutamicum*, *Corynebacterium crudilactis, Corynebacterium deserti, Corynebacterium efficiens*, *Corynebacterium callunae*, *Corynebacterium singulare*, *Corynebacterium halotolerans*, *Corynebacterium striatum*, *Corynebacterium ammoniagenes*, *Corynebacterium pollutisoli*, *Corynebacterium imitans*, *Corynebacterium testudinoris*, or *Corynebacterium flavescens.*

In the present disclosure, the "weakening" of a polypeptide activity includes both cases where the activity is decreased as compared to the endogenous activity or absent. The weakening may be used interchangeably with terms such as inactivation, deficiency, down-regulation, decrease, reduce, and attenuation.

The weakening may also include a case where the activity of the polypeptide itself is decreased or eliminated as compared to the activity of the polypeptide originally possessed by the microorganism by variation of the polynucleotide encoding the polypeptide, and the like, a case where the overall polypeptide activity level and/or concentration (expression level) in the cell is lower as compared to that of the natural strain by inhibition of the expression of the gene of the polynucleotide encoding the polypeptide or inhibition of translation into the polypeptide, a case where the polynucleotide is not expressed at all, and/or a case where the polypeptide activity is not exhibited even when the polynucleotide is expressed. The "endogenous activity" means the activity of a specific polypeptide originally possessed by the parent strain before the trait is changed or a wild-type or unmodified microorganism when the trait is changed by genetic variation due to natural or artificial factors. The endogenous activity may be used interchangeably with "activity before modification". The fact that the activity of a polypeptide is "inactivated, deficient, decreased, down-regulated, reduced, or attenuated" as compared to the endogenous activity means that the activity of a polypeptide is lowered as compared to the activity of a specific polypeptide originally possessed by the parent strain before the trait is changed or a wild-type or unmodified microorganism.

Such weakening of the activity of a polypeptide may be performed by any method known in the art, but the method is not limited thereto, and the weakening may be achieved by applying various methods well known in the art (for example, Nakashima N. et al., Bacterial cellular engineering by genome editing and gene silencing. Int J Mol Sci. 2014; 15(2):2773-2793, Sambrook et al., Molecular Cloning 2012, and the like).

Specifically, the weakening of the polypeptide activity in the present disclosure may be:
1) deletion of the entirety or a part of a gene encoding a polypeptide;
2) modification of an expression control region (or expression control sequence) to decrease the expression of a gene encoding a polypeptide;
3) modification of an amino acid sequence constituting the polypeptide to eliminate or weaken the activity of the polypeptide (for example, deletion/substitution/addition of one or more amino acids in an amino acid sequence);
4) modification of a gene sequence encoding a polypeptide to eliminate or weaken the activity of the polypeptide (for example, deletion/substitution/addition of one or more nucleic acid bases in a nucleic acid base sequence of the polypeptide gene to encode a polypeptide that has been modified to eliminate or weaken the activity of the polypeptide);
5) modification of a start codon of a gene transcript encoding a polypeptide or a base sequence encoding a 5'-UTR region;
6) introduction of an antisense oligonucleotide (for example, antisense RNA) that complementarily binds to the transcript of the gene encoding a polypeptide;
7) addition of a sequence complementary to a Shine-Dalgarno sequence in front of the Shine-Dalgarno sequence of a gene encoding a polypeptide in order to form a secondary structure to which ribosome cannot be attached;
8) addition of a promoter to be transcribed in the opposite direction to the 3' end of the open reading frame (ORF) of a gene sequence encoding a polypeptide (reverse transcription engineering, RTE); or
9) a combination of two or more selected from 1) to 8), but is not particularly limited thereto.

For example:
1) The deletion of a part or the entirety of the gene encoding a polypeptide may be removal of the entire polynucleotide encoding the endogenous polypeptide of interest in the chromosome, replacement with a polynucleotide in which some nucleotides are deleted or replacement with a marker gene.
2) The modification of the expression control region (or expression control sequence) may be deletion, insertion, non-conservative or conservative substitution, or occurrence of variation in the expression control region (or expression control sequence) due to a combination thereof or replacement with a sequence exhibiting weaker activity. The expression control region contains a promoter, an operator sequence, a sequence encoding a ribosome binding site, and a sequence controlling the termination of transcription and translation, but is not limited thereto.
3) and 4) The modification of an amino acid sequence or polynucleotide sequence may be deletion, insertion, or non-conservative or conservative substitution of an amino acid sequence of the polypeptide or a polynucleotide sequence encoding the polypeptide or occurrence of variation in the sequence due to a combination thereof or replacement with an amino acid sequence or polynucleotide sequence modified to exhibit weaker activity or an amino acid sequence or polynucleotide sequence modified to be inactive so that the activity of the polypeptide is weakened, but is not limited thereto. For example, the expression of a gene may be inhibited or weakened by introducing variation into the polynucleotide sequence and forming a stop codon, but the modification is not limited thereto.
5) The modification of a start codon of a gene transcript encoding a polypeptide or a base sequence encoding a 5'-UTR region may be, for example, substitution with a base sequence encoding another start codon having a lower polypeptide expression rate as compared to the endogenous start codon, but is not limited thereto.
6) For the introduction of an antisense oligonucleotide (for example, antisense RNA) that complementarily binds to the transcript of the gene encoding a polypeptide, reference may be made to documents, for example, Weintraub, H. et al., Antisense-RNA as a molecular tool for genetic analysis, Reviews-Trends in Genetics, Vol. 1(1) 1986.
7) The addition of a sequence complementary to a Shine-Dalgarno sequence in front of the Shine-Dalgarno sequence of a gene encoding a polypeptide in order to form a secondary structure to which ribosome cannot be attached may be to make mRNA translation impossible or to slow down the mRNA translation rate.
8) The addition of a promoter to be transcribed in the opposite direction to the 3' end of the open reading frame (ORF) of a gene sequence encoding a polypeptide (reverse transcription engineering, RTE) may be to weaken the activity by making an antisense nucleotide complementary to the transcript of the gene encoding a polypeptide.

In the present disclosure, the term "enhancement" of polypeptide activity means that the activity of a polypeptide is increased as compared to the endogenous activity. The enhancement may be used interchangeably with terms such as activation, up-regulation, overexpression, and increase. Here, activation, enhancement, up-regulation, overexpression, and increase may include both exhibiting activity that was not originally possessed and exhibiting improved activity as compared to the endogenous activity or activity before modification. The "endogenous activity" means the activity of a specific polypeptide originally possessed by the parent strain before the trait is changed or an unmodified microorganism when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of a polypeptide is improved as compared to the activity and/or concentration (expression level) of a specific polypeptide originally possessed by the parent strain before the trait is changed or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the polypeptide or in the amount of the product produced from the polypeptide.

For the enhancement of the activity of a polypeptide, various methods well known in the art may be applied, and the method is not limited as long as the activity of the polypeptide of interest can be enhanced as compared to that of the microorganism before being modified. Specifically, genetic engineering and/or protein engineering well known to those skilled in the art, which are routine methods of molecular biology, may be used, but the method is not limited thereto (for example, Sitnicka et al., Functional Analysis of Genes. Advances in Cell Biology. 2010, Vol. 2. 1-16; Sambrook et al., Molecular Cloning 2012; and the like).

Specifically, the enhancement of the activity of a polypeptide of the present disclosure may be:
1) increase in the intracellular copy number of a polynucleotide encoding a polypeptide;
2) replacement of a gene expression regulatory region on a chromosome encoding a polypeptide with a sequence exhibiting strong activity;
3) modification of a start codon of a gene transcript encoding a polypeptide or a base sequence encoding a 5'-UTR region;
4) modification of an amino acid sequence of a polypeptide to enhance the activity of the polypeptide;
5) modification of a polynucleotide sequence encoding a polypeptide to enhance the activity of the polypeptide (for example, modification of a polynucleotide sequence of the polypeptide gene to encode a polypeptide that has been modified to enhance the activity of the polypeptide);
6) introduction of a foreign polypeptide exhibiting the activity of a polypeptide or a foreign polynucleotide encoding the polypeptide;
7) codon optimization of a polynucleotide encoding a polypeptide;
8) analysis of the tertiary structure of a polypeptide to select and modification or chemical modification of the exposed site; or
9) a combination of two or more selected from 1) to 8), but is not particularly limited thereto.

More specifically:
1) The increase in the intracellular copy number of a polynucleotide encoding a polypeptide may be achieved by the introduction into a host cell of a vector which can be replicated and function independently of the host and to which a polynucleotide encoding the polypeptide is operably linked. Alternatively, the increase may be achieved by the introduction of one copy or two or more copies of a polynucleotide encoding the polypeptide into a chromosome of a host cell. The introduction into the chromosome may be performed by introducing a vector capable of inserting the polynucleotide into a chromosome of a host cell into the host cell, but is not limited thereto. The vector is as described above.
2) The replacement of a gene expression regulatory region (or expression control sequence) on a chromosome encoding a polypeptide with a sequence exhibiting strong activity may be, for example, deletion, insertion, non-conservative or conservative substitution, or occurrence of variation in a sequence due to a combination thereof or replacement with a sequence exhibiting stronger activity so that the activity of the expression control region is further enhanced. The expression control region is not particularly limited thereto, but may contain a promoter, an operator sequence, a sequence encoding a ribosome binding site, a sequence controlling the termination of transcription and translation, and the like. For example, the replacement may be to replace the original promoter with a strong promoter, but is not limited thereto.

Examples of known strong promoters include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, and yccA promoter, but are not limited thereto.

3) The modification of a start codon of a gene transcript encoding a polypeptide or a base sequence encoding a 5'-UTR region may be, for example, substitution with a base sequence encoding another start codon having a higher polypeptide expression rate as compared to an endogenous start codon, but is not limited thereto.

4) and 5) The modification of an amino acid sequence or polynucleotide sequence may be deletion, insertion, non-conservative or conservative substitution of an amino acid sequence of the polypeptide or a polynucleotide sequence encoding the polypeptide or occurrence of variation in the sequence due to a combination thereof or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further contain a selection marker for the confirmation of chromosome insertion. The selection marker is as described above.

6) The introduction of a foreign polypeptide exhibiting the activity of a polypeptide may be the introduction of a foreign polynucleotide encoding a polypeptide exhibiting activity the same as or similar to that of the polypeptide into a host cell. The foreign polynucleotide is not limited in its origin or sequence as long as it exhibits activity the same as or similar to that of the polypeptide. The introduction may be performed by appropriately selecting a known transformation method by those skilled in the art. As the introduced polynucleotide is expressed in a host cell, a polypeptide may be produced, and the activity thereof may be increased.

7) The codon optimization of a polynucleotide encoding a polypeptide may be codon optimization of an endogenous polynucleotide so as to increase transcription or translation in a host cell or codon optimization of a foreign polynucleotide so as to perform optimized transcription and translation in a host cell.

8) The analysis of the tertiary structure of a polypeptide to select and modification or chemical modification of the exposed site may be, for example, to determine a template protein candidate according to the degree of similarity of the sequence by comparing the sequence information of a polypeptide to be analyzed with a database storing the sequence information of known proteins, to confirm the structure based on this, and to select and modify or chemically modify the exposed portion to be modified or chemically modified.

Such enhancement of the polypeptide activity may be an increase in the activity or concentration expression level of the corresponding polypeptide based on the activity or concentration of the polypeptide expressed in a wild-type microbial strain or a microbial strain before being modified or an increase in the amount of a product produced from the polypeptide, but is not limited thereto.

In the microorganism of the present disclosure, partial or entire modification of a polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation and/or chemicals, but is not limited thereto. A method for modifying a part or the entirety of the gene may include a method using DNA recombination technology. For example, by introducing a nucleotide sequence or vector containing a nucleotide sequence homologous to the gene of interest into the microorganism to cause homologous recombination, a part or the entirety of the gene may be deleted. The introduced nucleotide sequence or vector may contain a dominant selection marker, but is not limited thereto.

In the microorganism of the present disclosure, the variant, polynucleotide, IMP, and the like are as described in the other aspects.

Still another aspect of the present disclosure provides a method for producing IMP, which includes culturing a *Corynebacterium stationis* strain comprising the variant of the present disclosure or the polynucleotide of the present disclosure in a medium.

The method for producing IMP of the present disclosure may include culturing a *Corynebacterium stationis* strain comprising the variant of the present disclosure or the polynucleotide of the present disclosure or the vector of the present disclosure in a medium.

In the present disclosure, the term "culture" means growing the *Corynebacterium stationis* strain of the present disclosure under appropriately controlled environmental conditions. The culture process of the present disclosure may be performed according to suitable medium and culture conditions known in the art. Such a culture process may be easily adjusted and used by those skilled in the art according to the selected strain. Specifically, the culture may be a batch type, continuous type, and/or fed-batch type, but is not limited thereto.

In the present disclosure, the term "medium" means a mixed substance containing nutrients required to culture the *Corynebacterium stationis* strain of the present disclosure as a main component, and the medium supplies nutrients, growth factors, and the like including water that are indispensable for survival and development. Specifically, as the medium and other culture conditions used for culture of the *Corynebacterium stationis* strain of the present disclosure, any one may be used without particular limitation as long as it is a medium used for common culture of microorganisms. The *Corynebacterium stationis* strain of the present disclosure may be cultured in a common medium containing proper carbon sources, nitrogen sources, phosphorus sources, inorganic compounds, amino acids and/or vitamins, and the like while controlling the temperature, pH, and the like under aerobic conditions.

Specifically, the culture medium for the strain of the genus *Corynebacterium* may be found in the document "Manual of Methods for General Bacteriology" by the American Society for Bacteriology (Washington, D.C., USA, 1981).

In the present disclosure, the carbon sources include carbohydrates such as glucose, saccharose, lactose, fructose, sucrose, and maltose; sugar alcohols such as mannitol and sorbitol, organic acids such as pyruvic acid, lactic acid, and citric acid; amino acids such as glutamic acid, methionine, and lysine; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (namely, molasses converted to reducing sugar) may be used, and appropriate amounts of other carbon sources may be used in various manners without limitation. These carbon sources may be used singly or in a combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in a combination of two or more, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, and the like may be used. In addition to these, amino acids, vitamins and/or suitable precursors, and the like may be contained. These components or precursors may be added to the medium batchwise or continuously, but the manner of addition is not limited thereto.

During the culture of the *Corynebacterium stationis* strain of the present disclosure, the pH of the medium may be adjusted by adding compounds such as ammonium hydroxide, potassium hydroxide, ammonia, phosphoric acid, and sulfuric acid to the medium in a proper manner. During the culture, foaming may be suppressed by using an antifoaming agent such as fatty acid polyglycol ester. Oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but the method for maintaining the state is not limited thereto.

In the culture of the present disclosure, the culture temperature may be maintained at 20°C to 45°C specifically 25°C to 40°C, and the strain may be cultured for about 10 to 160 hours, but the culture conditions are not limited thereto.

IMP produced through the culture of the present disclosure may be secreted into the medium or may remain in the cells.

The method for producing IMP of the present disclosure may further comprise a step of preparing the *Corynebacterium stationis* strain of the present disclosure, a step of preparing a medium for culture of the strain, or a combination of these (in any order), for example, prior to the culture step.

The method for producing IMP of the present disclosure may further include a step of recovering IMP from the medium according to the culture (the medium subjected to the culture) or from the *Corynebacterium stationis* strain of the present disclosure. The recovery step may be further included after the culture step.

The recovery may be to collect the IMP of interest by way of a suitable method known in the art according to the method for culturing the microorganism of the present disclosure, for example, a batch, continuous, or fed-batch culture method. For example, centrifugation, filtration, treatment with a crystallized protein precipitant (salting out), extraction, ultrasonic disintegration, ultrafiltration, dialysis, various forms of chromatography such as molecular-sieve chromatography (gel filtration), adsorption chromatography, ion-exchange chromatography, and affinity chromatography, HPLC, or a combination thereof may be used. The IMP of interest may be recovered from the medium or microorganism by way of a suitable method known in the art.

The method for producing IMP of the present disclosure may further include a purification step. The purification may be performed by way of a suitable method known in the art. In an example, when the method for producing IMP of the present disclosure includes both the recovery step and the purification step, the recovery step and the purification step may be performed continuously or discontinuously regardless of the order, or may be performed simultaneously or by being combined into one step, but the manner to perform the steps is not limited thereto.

In the method of the present disclosure, the variant, polynucleotide, vector, strain, IMP, and the like are as described in the other aspects.

Still another aspect of the present disclosure is to provide a composition for IMP production, which comprises a *Corynebacterium stationis* strain comprising the variant of the present disclosure, a polynucleotide encoding the variant, a vector comprising the polynucleotide, or the polynucleotide of the present disclosure; a medium in which this *Corynebacterium stationis* strain has been cultured; or a combination of two or more of these.

The composition of the present disclosure may further comprise arbitrary suitable excipients to be commonly used in compositions for IMP production. Such excipients may be, for example, a preservative, a wetting agent, a dispersing agent, a suspending agent, a buffering agent, a stabilizer, or an isotonic agent, but are not limited thereto.

In the composition of the present disclosure, the variant, polynucleotide, vector, strain, medium, IMP, and the like are as described in the other aspects.

### [Mode for Invention]

Hereinafter, the present disclosure will be described in more detail by way of Examples. However, the following Examples are only preferred embodiments for illustrating the present disclosure. Meanwhile, technical matters not described in the present specification can be sufficiently understood and easily implemented by those skilled in the technical field of the present disclosure or similar technical fields.

### Example 1. Construction of vector for bifunctional pyr operon transcriptional regulator/uracil phosphoribosvltransferase variant expression in microorganism

In order to explore the effect of a variant (P108S; SEQ ID NO: 1), in which proline at position 108 of a bifunctional pyr operon transcriptional regulator/uracil phosphoribosyltransferase amino acid sequence (SEQ ID NO: 3) was substituted with serine, on IMP production, a vector for the construction of a strain expressing the variant was constructed as follows using the plasmid pDCM2 (Korean Publication No. 10-2020-0136813) for the insertion and replacement of genes in the chromosome of Corynebacterium.

PCR was performed using gDNA (genomic DNA) of wild-type *Corynebacterium stationis* ATCC6872 as a template and a pair of primers having sequences of SEQ ID NOs: 5 and 6, and a pair of primers having sequences of SEQ ID NOs: 7 and 8. Overlapping PCR was performed again using the mixture of two fragments obtained above as a template and a pair of primers having sequences of SEQ ID NO: 5 and SEQ ID NO: 8 to obtain a fragment. The PCR was performed as follows: denaturation at 94°C for 5 minutes; 30 cycles of 94°C for 30 seconds, 55°C for 30 seconds, and 72°C for 1 minute 30 seconds; and 72°C for 5 minutes. The pDCM2 vector was treated with Smal, and the PCR product obtained above was fusion cloned thereinto. Fusion cloning was performed using an In-Fusion^{®} HD cloning kit (Clontech). The obtained plasmid was named pDCM2-pyrR(P108S).

### Example 2: Evaluation of IMP producing ability of microorganism expressing bifunctional pyr operon transcriptional regulator/uracil phosphoribosvltransferase variant

### 2-1. Construction of strain for expressing bifunctional pyr operon transcriptional regulator/uracil phosphoribosyltransferase variant

The vector constructed in Example 1 was transformed into *Corynebacterium stationis* CJI2332 strain (KCCM12277P, KR 10-1950141 B1).

The strain into which the variant was introduced was selected from the strains in which homologous recombination took place using SEQ ID NOs: 9 and 10. The selected strain was named CJI2332_pyrR_P108S.

### 2-2. Comparison of IMP producing ability of strains expressing bifunctional pyr operon transcriptional regulator/uracil phosphoribosyltransferase variant

The IMP producing ability was analyzed through flask fermentation titer evaluation of each strain constructed in Example 2-1 and a control parent strain.

First, each strain was inoculated into a test tube with a diameter of 18 mm containing 2 mL of seed medium and shake-cultured for 24 hours at 30°C to be used as a seed culture solution. Into a 250 mL corner-baffle flask containing 29 mL of the following production medium (24 mL of main medium + 5 mL of separate sterilization medium), 2 mL of the seed culture solution was inoculated and cultured at 170 rpm for 72 hours at 30°C. After completion of the culture, the IMP producing ability was measured by HPLC. The IMP concentration in the culture solution for each of the tested strains and the rate of increase in the IMP concentration are as presented in Table 1 below.

### <Seed medium (pH 7.5)>

Glucose 1%, peptone 1%, beef extract 1%, yeast extract 1%, sodium chloride 0.25%, adenine 100 mg/L, and guanine 100 mg/L (based on 1 liter of distilled water)

### <Production medium - main medium (pH 7.5)>

Yeast extract 0.2%, sodium glutamate 0.1%, magnesium sulfate 1.2%, calcium chloride 0.01%, iron sulfate 20 mg/L, manganese sulfate 20 mg/L, zinc sulfate 20 mg/L, copper sulfate 5 mg/L, L-cysteine 23 mg/L, beta-alanine 24 mg/L, nicotinic acid 8 mg/L, biotin 45 µg/L, thiamine hydrochloride 5 mg/L, adenine 30 mg/L, glucose 2.55%, and fructose 1.45% (based on 1 liter of distilled water)

### <Production medium - separate sterilization medium (pH 7.5)>

Phosphoric acid (85%) 2.3%, potassium hydroxide (45%) 2.55% (based on 1 liter of distilled water)

The experiment was repeated 3 times, and the average values of the analysis results thereof are presented in Table 1 below.

**[Table 1]**

| Comparison of IMP producing ability | | |
|---|---|---|
| Strain | IMP concentration (g/L) | Rate of increase in IMP concentration (%) |
| CJI2332 | 1.79 | - |
| CJI2332_pyrR_P108S | 2.29 | 27.9 |

As presented in Table 1, the CJI2332_pyrR_P108S strain exhibited IMP producing ability increased by 27.9% as compared to that of the control group.

## Claims

1. A bifunctional pyr operon transcriptional regulator/uracil phosphoribosyltransferase variant consisting of an amino acid sequence represented by SEQ ID NO: 1, wherein proline, which is an amino acid corresponding to position 108 of SEQ ID NO: 3, is substituted with serine.

2. A polynucleotide encoding the variant according to claim 1.

3. A *Corynebacterium stationis* strain comprising the variant according to claim 1 or a polynucleotide encoding the variant.

4. The strain according to claim 3, which has increased IMP producing ability as compared to *Corynebacterium stationis* comprising a polypeptide of SEQ ID NO: 3 or a polynucleotide encoding the polypeptide.

5. A method for producing IMP, the method comprising culturing a *Corynebacterium stationis* strain comprising the variant according to claim 1 or a *Corynebacterium stationis* strain comprising a polynucleotide encoding the variant in a medium.

## Patentansprüche

1. Bifunktionelle pyr-Operon-Transkriptionsregulator-/Uracilphosphoribosyltransferase-Variante, bestehend aus einer Aminosäuresequenz, dargestellt durch SEQ ID NO: 1, worin Prolin, das eine Aminosäure ist, die Position 108 von SEQ ID NO: 3 entspricht, durch Serin ersetzt ist.

2. Polynucleotid, das für eine Variante nach Anspruch 1 kodiert.

3. Corynebacterium-stationis-Stamm, umfassend eine Variante nach Anspruch 1 oder ein für die Variante kodierendes Polynucleotid.

4. Stamm nach Anspruch 3, der eine erhöhte IMP-produzierende Fähigkeit verglichen mit Corynebacterium stationis, umfassend ein Polypeptid von SEQ ID NO: 3 oder ein für das Polypeptid kodierendes Polynucleotid, aufweist.

5. Verfahren zur Produktion von IMP, wobei das Verfahren das Kultivieren eines Corynebacterium-stationis-Stamms, umfassend eine Variante nach Anspruch 1, oder eines Corynebacterium-stationis-Stamms, umfassend ein für die Variante kodierendes Polynucleotid, in einem Medium umfasst.

## Revendications

1. Variant bifonctionnel de régulateur transcriptionnel d'opéron pyr/uracil phosphoribosyltransférase consistant en une séquence d'acides aminés représenté par SEQ ID NO: 1, dans lequel la proline, qui est un acide aminé correspondant à la position 108 de SEQ ID NO : 3, est substituée par de la sérine.

2. Polynucléotide codant pour le variant selon la revendication 1.

3. Souche de *Corynebacterium stationis* comprenant le variant selon la revendication 1 ou un polynucléotide codant pour le variant.

4. Souche selon la revendication 3, qui présente une capacité de production d'IMP accrue par rapport au *Corynebacterium stationis* comprenant un polypeptide de SEQ ID NO : 3 ou un polynucléotide codant pour le polypeptide.

5. Procédé de production d'IMP, le procédé comprenant la culture d'une souche de *Corynebacterium stationis* comprenant le variant selon la revendication 1 ou une souche de *Corynebacterium stationis* comprenant un polynucléotide codant pour le variant dans un milieu.
